# EUROPEAN PATENT APPLICATION

(11) **EP 4 400 029 A1**
(43) Date of publication of application: **17.07.2024**
(21) Application number: 22867105.3
(22) Date of filing: 27.07.2022
(51) Int. Cl.: A61B 1/06, A61B 1/045

(54) **LIGHT SOURCE DEVICE AND ENDOSCOPE SYSTEM**

(30) Priority: 09.09.2021 JP 2021147112
(71) Applicant: HOYA CORPORATION, Shinjuku-ku Tokyo 160-8347 (JP)
(72) Inventor: HAYASHI, Yoshihiro, Tokyo 160-8347 (JP)
(74) Representative: Schaumburg und Partner Patentanwälte mbB
(86) International application number: PCT/JP2022/028964
(87) International publication number: WO 2023/037783

(57) **Abstract**

The present disclosure proposes a light source device that generates illumination light to be applied to an object in order to enable light amount control to be executed quickly in response to a change during a rolling shutter period (pixel readout period) while avoiding the occurrence of distortion and artifacts caused by a rolling shutter of an image sensor, and includes a plurality of semiconductor light emitting elements that emit pieces of light having different wavelength bands, and a control unit that controls a light emission profile of the plurality of semiconductor light emitting elements and drives the plurality of semiconductor light emitting elements, in which the control unit controls the light emission profile of the plurality of semiconductor light emitting elements such that main light emission is performed in a pseudo global exposure period of an image sensor and preliminary light emission is performed in at least a part of a pixel readout period of the image sensor, and a light emission level of the preliminary light emission is lower than a light emission level of the main light emission

## Description

### Technical Field

The present disclosure relates to a light source device and an endoscope system.

### Background Art

In a normal endoscope device equipped with a rolling shutter type image sensor, a light source is turned off in a valid pixel readout period (rolling shutter period) of the image sensor, and the light source is turned on in other periods (pseudo global exposure period) (pulse light emission control is performed), thereby executing pseudo global exposure and avoiding the occurrence of an undesirable phenomenon caused by the rolling shutter, for example, distortion or artifacts.

On the other hand, when the light source is completely turned off during the rolling shutter period, a light amount becomes insufficient depending on an object (observation target site), and a favorable image cannot be acquired. For example, Patent Literatures 1 to 3, and the like, disclose light source control in which a part of the rolling shutter period is included in a pulse light emission period in order to solve having an insufficient light amount.

### Citation List

### Patent Literature

Patent Literature 1: JP 2018-182580 A
Patent Literature 2: JP 5379932 B1
Patent Literature 3: JP 6239220 B1

### Summary of Invention

### Technical Problem

However, when the light source control as described in Patent Literatures 1 to 3 is executed, brightness unevenness, lateral stripes, and the like, of a screen occur due to an exposure period difference for each line in adjacent frames. There is a problem that the brightness unevenness and the lateral stripes move up and down on a display screen due to a change in the pulse light emission period for each frame and obstruct viewing. In a case where offset light emission is performed during the rolling shutter period in order to solve the insufficient light amount, when the offset light emission becomes strong to some extent, an unnatural image such as a long-time exposure image and a high-speed exposure image subjected to double exposure is generated (occurrence of artifacts and scanning line noise (distortion)).

In the techniques according to Patent Literatures 1 to 3, an event that has changed during the turn-off period (rolling shutter period) is not observed in the readout in that period, and can be observed only in the next readout frame exposed by the next pulse light emission. Thus, there is a risk that perforation or halation will occur in an image when a distal tip of an endoscope suddenly approaches an object. In order to suppress the risk of occurrence of such perforation or halation, it is necessary to promptly ascertain a change in brightness of a screen due to a sudden approach and immediately reflect the change in brightness in light amount control.

The present disclosure has been made in view of such a situation, and proposes a technique capable of executing light amount control quickly in response to a change during a rolling shutter period (pixel readout period) while avoiding the occurrence of distortion and artifacts caused by a rolling shutter of an image sensor.

### Solution to Problem

In order to solve the above problem, according to the present embodiment, there is provided a light source device that generates illumination light to be applied to an object, the light source device including a plurality of semiconductor light emitting elements that emit pieces of light having different wavelength bands; and a control unit that controls a light emission profile of the plurality of semiconductor light emitting elements and drives the plurality of semiconductor light emitting elements, in which the control unit controls the light emission profile of the plurality of semiconductor light emitting elements such that main light emission is performed in a pseudo global exposure period of an image sensor and preliminary light emission is performed in at least a part of a pixel readout period of the image sensor, and a light emission level of the preliminary light emission is lower than a light emission level of the main light emission.

According to the present embodiment, there is provided an endoscope system in which an endoscope is inserted into an observation target and an image of an object is acquired, the endoscope system including a plurality of semiconductor light emitting elements that emit pieces of light having different wavelength bands; an image sensor that irradiates the object with illumination light and detects reflected light from the object to generate an image signal; a processor that processes the image signal to generate the image of the object and displays the image on a monitor; a main control unit that generates a control signal for controlling a light emission profile of the plurality of semiconductor light emitting elements on the basis of the image signal; and a light source control unit that receives the control signal from the main control unit and drives the plurality of semiconductor light emitting elements with a drive signal according to the light emission profile, in which the main control unit controls the light emission profile of the plurality of semiconductor light emitting elements such that main light emission is performed in a pseudo global exposure period of the image sensor and preliminary light emission is performed in at least a part of a pixel readout period of the image sensor, and a light emission level of the preliminary light emission is lower than a light emission level of the main light emission.

Features related to the present disclosure will become apparent from the description of the present specification and the accompanying drawings. The present disclosure is achieved and implemented by elements and combinations of various elements and by modes of the following detailed description and the appended claims.

It is to be understood that the description in this specification is merely exemplary and is not intended to limit the significance of the claims or the application in any way.

### Advantageous Effects of Invention

According to the present disclosure, it is possible to execute light amount control quickly in response to a change during a rolling shutter period (pixel readout period) while avoiding the occurrence of distortion and artifacts caused by a rolling shutter of an image sensor.

### Brief Description of Drawings

Fig. 1 is a diagram illustrating an overall external appearance example of an endoscope system according to the present embodiment.
Fig. 2 is a diagram illustrating a schematic internal configuration example of the endoscope system according to the present embodiment.
Fig. 3 is a diagram illustrating an internal configuration example of a light source device 201 provided inside a processor 200.
Fig. 4 is a diagram illustrating spectra (wavelength characteristics) of LEDs 2011 to 2015. Fig. 5 is a diagram illustrating characteristics of illumination light (light illuminating an observation site) generated by transmitting light from each LED through cross prisms 2017 and 2018.
Fig. 6 is a diagram illustrating a configuration example of a light source using LEDs having different light distributions.
Fig. 7 is a graph of an emitted light amount/current ratio of each LED.
Fig. 8 is a diagram illustrating a valid pixel region and an invalid region of a rolling shutter type image sensor using a CMOS sensor as an example.
Fig. 9 is a diagram illustrating a state (example) of a general dimming control process and an observation image (example) acquired in each frame.
Fig. 10 is a diagram illustrating a state (example) of a dimming control process according to Control Example 1 of the present embodiment and an observation image (example) acquired in each frame.
Fig. 11 is a diagram illustrating a state (example) of a dimming control process according to Control Example 2 of the present embodiment and an observation image (example) acquired in each frame.
Fig. 12 is a diagram illustrating a state (example) of a dimming control process according to Control Example 3 of the present embodiment and an observation image (example) acquired in each frame.
Fig. 13 is a diagram illustrating a state (example) of a dimming control process according to Control Example 4 of the present embodiment and an observation image (example) acquired in each frame.
Fig. 14 is a flowchart for describing a dimming control process according to Control Examples 1 and 2 of the present embodiment.
Fig. 15 is a flowchart for describing a dimming control process according to Control Examples 3 and 4 of the present embodiment.

### Description of Embodiments

Hereinafter, embodiments of the present disclosure will be described with reference to the drawings. In the following description, an endoscope system will be described as an embodiment of the present disclosure.

An observation target site in the endoscope system is, for example, respiratory organs or digestive organs. Examples of the respiratory organs include the lungs, the bronchus, the ears, the nose, and the throat. Examples of the digestive organs include the large intestine, the small intestine, the stomach, the esophagus, the duodenum, the uterus, and the bladder. In a case of observing the target sites as described above, it is more valid to utilize an image in which a specific biological structure is emphasized.

### <Configuration of endoscope system>

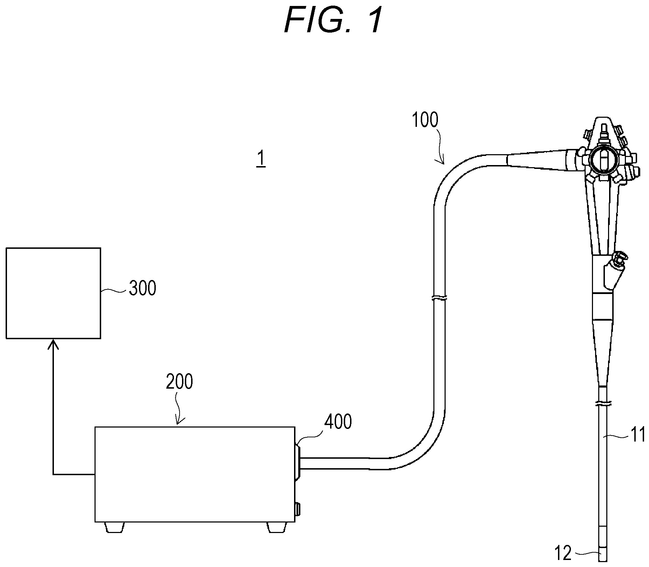

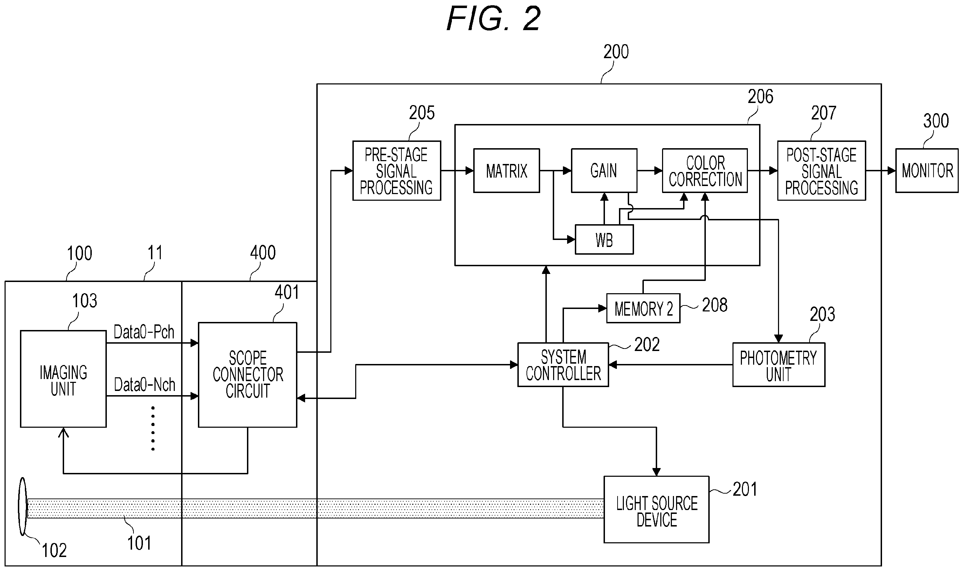

Fig. 1 is a diagram illustrating an example of an overall external appearance of an endoscope system of the present embodiment, and Fig. 2 is a diagram illustrating a schematic internal configuration example of the endoscope system of the present embodiment. An endoscope system 1 includes an endoscope device (electronic scope) 100, a processor 200, and a monitor 300. Note that a scope connector (which may hereinafter be simply referred to as a "connector") 400 including a connector circuit according to a feature of the present embodiment is provided at a processor-side end portion of the endoscope device 100.

The endoscope device 100 includes an elongated tubular insertion portion 11 to be inserted into an object. The endoscope device 100 includes a light carrying bundle (LCB) 101 for guiding irradiation light from a light source device 201 that will be described later, a light distribution lens 102 provided at an emission end of the LCB 101, an imaging unit 103 that receives return light from an irradiated portion (observation site) via an objective lens (not illustrated), a driver signal processing circuit 105 that drives the imaging unit 103, and a first memory 106.

The irradiation light from the light source device 201 enters the LCB 101 and propagates by repeating total reflection in the LCB 101. The irradiation light (illumination light) propagating in the LCB 101 is emitted from the emission end of the LCB 101 disposed in a distal tip portion 12 of the insertion portion 11 and irradiates the observation site through the light distribution lens 102. The return light from the irradiated portion forms an optical image at each pixel on a light receiving surface of the imaging unit 103 via the objective lens.

The imaging unit 103 is disposed in the distal tip portion 12 of the insertion portion 11 and can use a complementary metal oxide semiconductor (CMOS) image sensor which is a rolling shutter type image sensor. The imaging unit 103 accumulates optical images (return light from a living tissue) formed at each pixel on the light receiving surface, as electric charge corresponding to a light amount and generates and outputs image signals of R, G, and B. Note that the imaging unit 103 is not limited to the CMOS image sensor and may be replaced with another type of imaging device as long as it is based on the rolling shutter type. A signal output from the imaging unit 103 is processed by a scope connector circuit 401 provided in the scope connector 400 as will be described later.

The processor 200 is a device that integrally includes a signal processing device that processes a signal from the endoscope device 100 and a light source device that irradiates, via the endoscope device 100, a body cavity where natural light cannot reach. In another embodiment, the signal processing device and the light source device may be provided separately. The processor 200 includes a light source device 201, a system controller 202, a photometry unit 203, a pre-stage signal processing circuit 205, a color conversion circuit 206, a post-stage signal processing circuit 207, and a second memory 208.

The processor 200 may include an operation panel (not illustrated). There are various forms in a configuration of the operation panel. Examples of a specific configuration of the operation panel include a hardware key for each function mounted on a front surface of the processor 200, a touch panel type graphical user interface (GUI), a combination of the hardware key and the GUI, and the like. An operator (surgeon) can perform a mode switching operation that will be described later with the operation panel.

The photometry unit 203 acquires luminance information of an image signal obtained through imaging from a gain circuit included in the color conversion circuit 206, compares the acquired luminance information with a predetermined appropriate luminance value (for example, information of the appropriate luminance value may be stored in advance in an internal memory (not illustrated) of the photometry unit 203) and notifies the system controller 202 of a comparison result (whether a current luminance value is appropriate, higher, or lower).

The system controller 202 executes various programs stored in a memory (not illustrated) and integrally controls the entire endoscope system 1. The system controller 202 controls operations and timings of various circuits in the processor 200 by using a control signal to perform processing suitable for the endoscope device 100 connected to the processor 200. The system controller 202 may be connected to the above-described operation panel.

The system controller 202 receives the comparison result with the appropriate luminance value from the photometry unit 203, determines whether to maintain current exposure (exposure), whether to increase the exposure (including a level value to increase), or whether to decrease the exposure (including a level value to decrease) and outputs the comparison result to the light source device 201 as an exposure control signal.

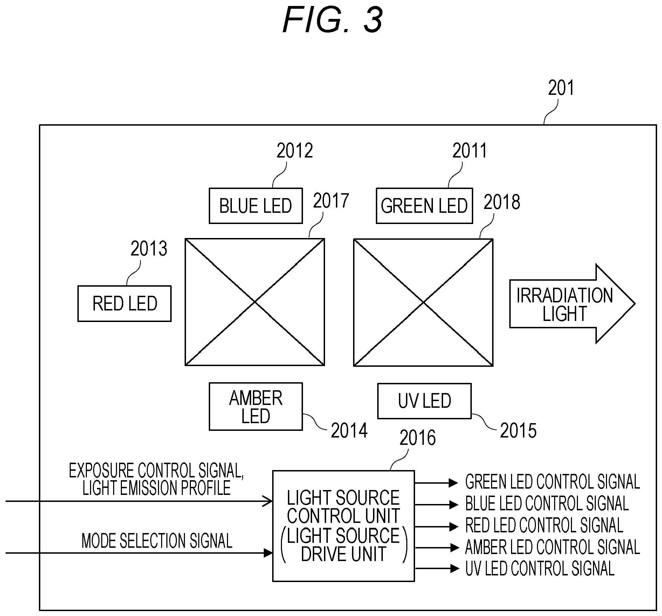

The system controller 202 changes each operation of the endoscope system 1 and parameters for each operation in accordance with an operator's instruction input from the operation panel. For example, when the operator selects an observation mode with the operation panel (mode switching operation), the system controller 202 outputs a mode selection signal for causing a light source corresponding to the observation mode to emit light to the light source device 201. As will be described later, as the light source device 201, for example, a plurality of light emitting diodes (LEDs) that emit light with different wavelength bands may be used (see Fig. 3). When the operator selects an observation mode (for example, a normal observation mode, a special light observation mode, a SatO2 mode, and the like) by operating a mode selection switch provided in the processor 200, for example, the system controller 202 generates a mode selection signal corresponding to the selected mode and supplies the mode selection signal to a light source control unit 2016 of the light source device 201 (see Fig. 3). On the basis of the mode selection signal, the light source control unit 2016 determines a combination of LEDs that will emit light and their intensity and light amounts (for example, a combination, and the like, of light emitting LEDs corresponding to the mode selection signal are stored in advance in an internal memory (not illustrated)) and outputs a necessary LED control signal from the LEDs 2011 to 2015. When each of the LEDs 2011 to 2015 emits light of each wavelength band based on the LED control signal supplied from the light source control unit 2016, the emitted light is synthesized by a cross prism to generate irradiation light (synthesized light).

The endoscope device 100 and the processor 200 may perform data communication using a wired electric communication method or an optical wireless communication method.

As illustrated in Fig. 2, the endoscope device 100 and the processor 200 are connected via the scope connector 400. The scope connector 400 includes an LCB configuring a part of the LCB 101 continuing from the processor 200 to the endoscope device 100 and a scope connector circuit 401. The scope connector circuit 401 is provided in the scope connector 400 in the present embodiment, but is not necessarily provided in the scope connector 400. For example, a circuit corresponding to the scope connector circuit 401 may be provided in a connector unit on the processor 200 side or inside the processor 200.

### <Internal configuration example of light source device 201>

Fig. 3 is a diagram illustrating an internal configuration example of the light source device 201 provided inside the processor 200, for example.

The light source device 201 includes a green LED 2011 that emits green light, a blue LED 2012 that emits blue light, a red LED 2013 that emits red light, an amber LED 2014 that emits amber light, a UV LED 2015 that emits UV light, a light source control unit 2016 that controls light emission of the LEDs 2011 to 2015, and cross prisms 2017 and 2018.

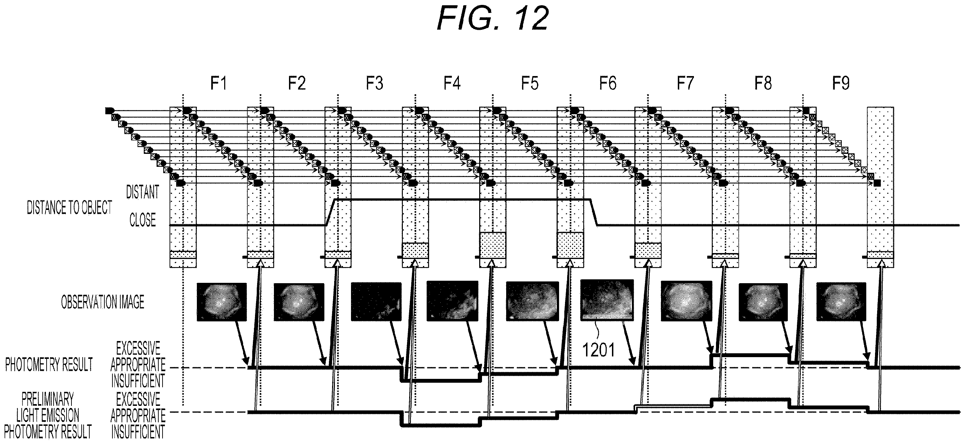

When the light source control unit 2016 receives the exposure control signal from the system controller 202, the light source control unit 2016 changes a light emission profile of each LED and performs exposure adjustment (light amount adjustment) by controlling a light emission period and an applied current value of each LED that is currently emitting light (a combination of LEDs that will emit light is determined depending on the observation mode) (see Fig. 12 that will be described later). For example, after changing the light emission profile by one step, the light source control unit 2016 determines whether to change the light emission profile again and perform exposure adjustment on the basis of an exposure control signal determined from a photometry result (a comparison result with the appropriate luminance value) from the photometry unit 203.

The light source control unit 2016 determines a combination of LEDs that will emit light on the basis of a mode selection signal indicating an observation mode selected by the operator. In a light emission start stage, the light source control unit 2016 controls light emission of each LED on the basis of, for example, a predetermined light emission profile (a default light emission period and a drive current value), and thereafter, performs exposure adjustment as described above.

### <Each LED light source>

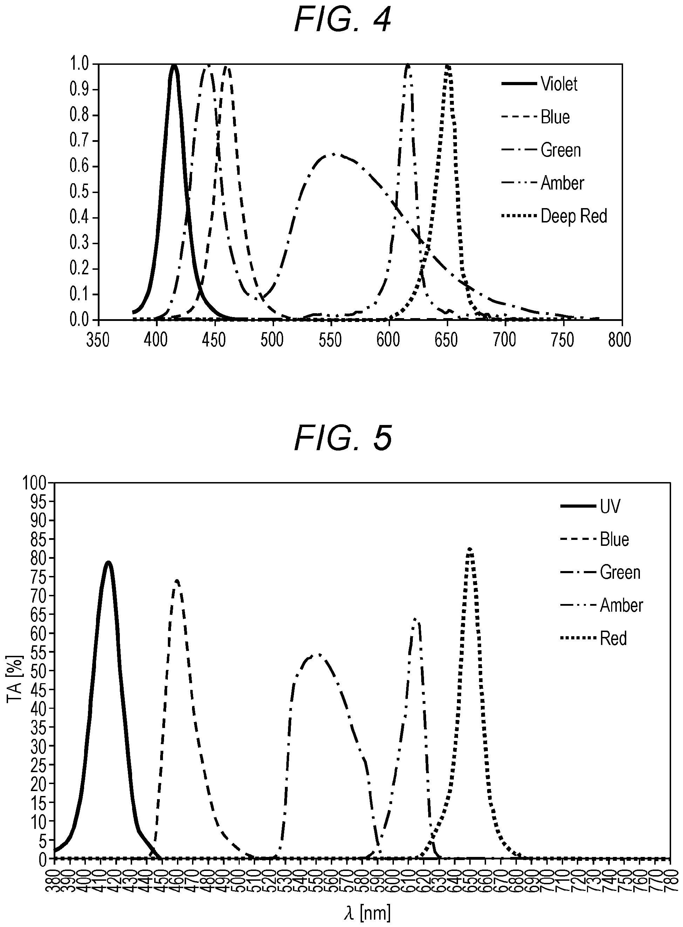

Fig. 4 is a diagram illustrating spectra (wavelength characteristics) of the LEDs 2011 to 2015. Fig. 5 is a diagram illustrating characteristics of illumination light (light illuminating an observation site) generated by transmitting light from each LED through the cross prisms 2017 and 2018.

A transmission wavelength band of the green LED 2011 is 540 nm to 575 nm, a peak wavelength is 550 nm, and a half-value width is 30 nm. A phosphor is provided in the green LED 2011, and the phosphor emits light in a transmission wavelength range of about 400 nm to 780 nm as illustrated in Fig. 4. In other words, although white light is substantially emitted by the green LED and the phosphor, this white light is an intermediate product, and as will be described later, the transmission wavelength band is narrowed by the cross prism 2018, and an observation site is irradiated with green light. A transmission wavelength band of the blue LED 2012 is 460 nm to 490 nm, a peak wavelength is 456 nm, and a half-value width is 21 nm. A transmission wavelength band of the red LED 2013 is 630 nm to 1000 nm, a peak wavelength is 650 nm, and a half-value width is 20 nm. A transmission wavelength band of the amber LED 2014 is 600 nm to 615 nm, a peak wavelength is 613 nm, and a half-value width is 19 nm. A transmission wavelength band of the UV LED 2015 is 385 nm to 425 nm, a peak wavelength is 405 nm, and a half-value width is 14 nm.

The light (the white light as an intermediate product, the blue light, the red light, the amber light, or the UV light) emitted from each of the LEDs 2011 to 2015 including the green LED 2011 in which the phosphor is provided is transmitted through the cross prisms 2017 and 2018 to become light having the characteristics illustrated in Fig. 5, and is applied to the observation site. Specifically, the transmission wavelength band of the white light emitted from the green LED 2011 + the phosphor is limited by the cross prism 2018, so that the white light becomes green light with 520 nm to 595 nm. The blue light emitted from the blue LED 2012 becomes blue light with 440 nm to 500 nm by the cross prisms 2017 and 2018. The red light emitted from the red LED 2013 becomes red light with 620 nm to 630 nm by the cross prisms 2017 and 2018. The amber light emitted from the amber LED 2014 becomes amber light with 580 nm to 630 nm by the cross prisms 2017 and 2018. The UV light emitted from the UV LED 2015 becomes UV light with 380 nm to 450 nm by the cross prism 2018.

### <Correction of linearity difference of each LED>

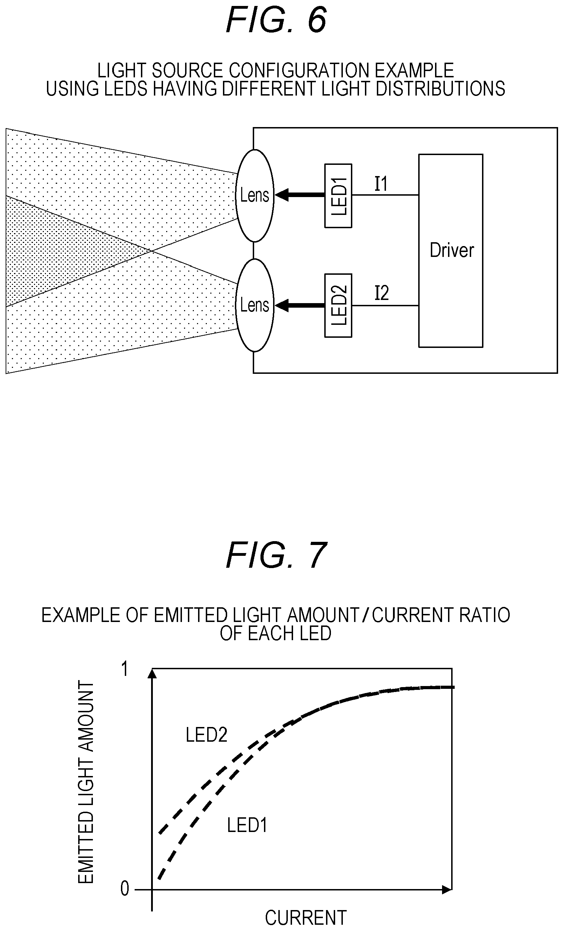

In a case where the light source device 201 includes a plurality of LEDs, not only wavelengths of light emitted from the LEDs 2011 to 2015 but also light distributions (light intensity distributions in respective directions) may be different (see Fig. 6: configuration example of light source using LEDs having different light distributions), and a color or a light distribution of emitted light from each of the LEDs 2011 to 2015 may change. Depending on the type of the LED, when a forward voltage is lowered in order to lower the drive current value, a drive current value rapidly decreases and the LED does not emit light, and thus there is a case where the drive current value cannot be greatly lowered. In order to cope with such a situation, it is necessary to dynamically correct a difference between linearities of the emitted light amount/current ratios of the respective LEDs 2011 to 2015 in accordance with drive current control for the LEDs 2011 to 2015.

However, a process of dynamically correcting the difference in linearity is complicated, and thus it is preferable to determine a drive current value in advance such that there is no difference in linearity. Therefore, in the present embodiment, a correction table for correcting the linearity of the emitted light amount/current ratio is prepared in advance, and a drive current value for each of the LEDs 2011 to 2015 is determined by using the correction table. Fig. 7 is a graph illustrating an emitted light amount/current ratio of each LED. Fig. 7 illustrates a relationship between only two LEDs (the LED 1 and the LED 2) as an example, but the same applies to the case of using five LEDs 2011 to 2015 as described in the present embodiment. The relationship between the emitted light amount/current ratio of each LED as illustrated in Fig. 7 may be acquired by measuring each LED in advance. Thus, a correction table having a reciprocal of the relationship between the emitted light amount/current ratios as a correction parameter is provided in advance (stored in a memory) as a correction value, and the light source control unit 2016 multiplies a correction parameter corresponding to a desired emitted light amount (target emitted light amount obtained through exposure adjustment) to calculate a corrected drive current value and drives each LED. This makes it possible to appropriately control the linearity of the emitted light amount/current ratio even in a case where wavelengths or light distributions of emitted light of the respective LEDs are different.

### <Configuration example of imaging surface of image sensor>

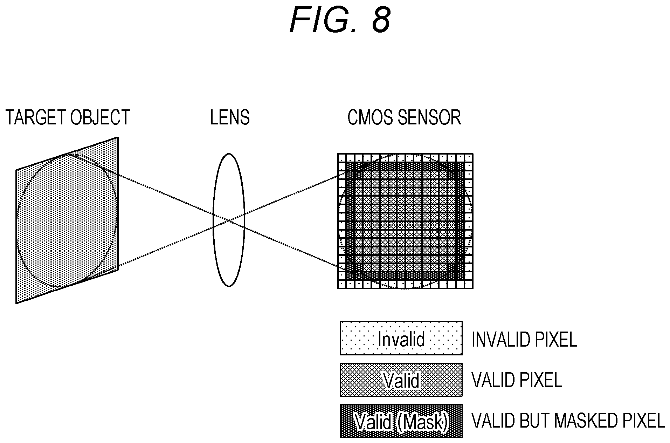

Fig. 8 is a diagram illustrating a valid pixel region and an invalid pixel region of a rolling shutter type image sensor using a CMOS sensor as an example. The CMOS sensor includes a valid pixel region in which imaging can be performed and an invalid region in which imaging cannot be performed. A part (peripheral portion) of the valid pixel region is masked and is a region in which an image signal cannot be substantially acquired. In a case where imaging is performed by using such an image sensor (in a case of global exposure), various phenomena (features) appear in a captured image. Note that, in the present embodiment, a period during which an image is not displayed on a screen is the global exposure period, but an idea of the present embodiment is not limited to this case.

### <General dimming control process>

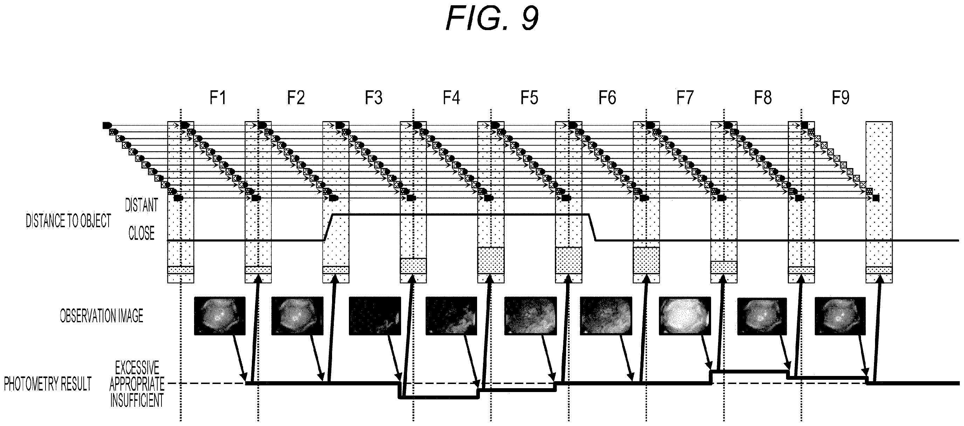

Fig. 9 is a diagram illustrating a state (example) of a general dimming control process and an observation image (example) acquired in each frame.

As illustrated in Fig. 9, it can be seen that frames F1 and F2 are observed with an appropriate light amount (a photometry result is appropriate). In a pseudo global exposure period between the frame F2 and a frame F3, since the endoscope distal tip is suddenly moved away from an object, an amount of irradiation light to the object is insufficient, and an observation image of the frame F3 is dark. A light amount at the time of acquiring the observation image of the frame F3 is dominated by a light amount from light emission (strong light emission: also referred to as main light emission) in the pseudo global exposure period between the previous frame (frame F2) and the current frame (frame F3). Therefore, in a case where a distance to the object changes after the pseudo global exposure period, light emission (dimming control) following the change in the distance is performed (reflected) in the next frame (frame F4). Thus, in the pseudo global period between the frame F3 and the frame F4, the dimming control is executed stepwise on the basis of the photometry result (light emission control (change of a light emission profile)) is performed stepwise according to the lapse of time (in order of the frames F4, F5, and F6), an appropriate light amount is determined (light amount increase control), and the object is irradiated with an appropriate amount of light. An observation image with an appropriate light amount can be acquired in the frame F5 after the endoscope distal tip is moved away from the object (after being moved away between F2 and F3).

In the pseudo global exposure period between the frame F5 and the frame F6, since a distance between the endoscope distal tip and the object is the same as that in the previous frame F5, light emission is performed in the same profile as that of light emission in the pseudo global exposure period between the frame F4 and the frame F5. Therefore, the observation image obtained in the frame F6 is the same as the observation image obtained in the frame F5. However, in practice, since the endoscope distal tip is rapidly approaching the object during a pixel readout period (rolling shutter period) for the frame F6, a light emission amount based on the latest light emission profile is not suitable for the next frame observation, and a light amount becomes excessive, and halation occurs in an image of the frame F7. This is because a light emission amount (light emission profile) in the pseudo global period between the frame F6 and the frame F7 is determined on the basis of a photometry result of the image acquired in the frame F6 (a photometry result of the observation image of the frame F6 is appropriate). Only after the photometry result of the observation image of the frame F7 due to the excessive light emission amount is obtained, the excessive light emission amount due to sudden approach (including collision) to the object is corrected. That is, the light emission profile is changed for the first time in a pseudo global exposure period between the frame F7 and a frame F8 (even when a light amount is reduced, the light emission profile is changed stepwise in the same manner as when the light amount is increased.).

As described above, according to the general dimming control process, in a case where the endoscope distal tip suddenly approaches the object, the dimming control (amount reduction) is delayed by one frame. In the case of sudden approach, a light amount becomes excessive, and thus halation occurs in the observation image, and it is very difficult for the operator to perform observation.

The present embodiment discloses a process of correcting the above disadvantage of general dimming control (delay of light emission profile change).

### <Dimming control process according to present embodiment>

### (i) Control Example 1

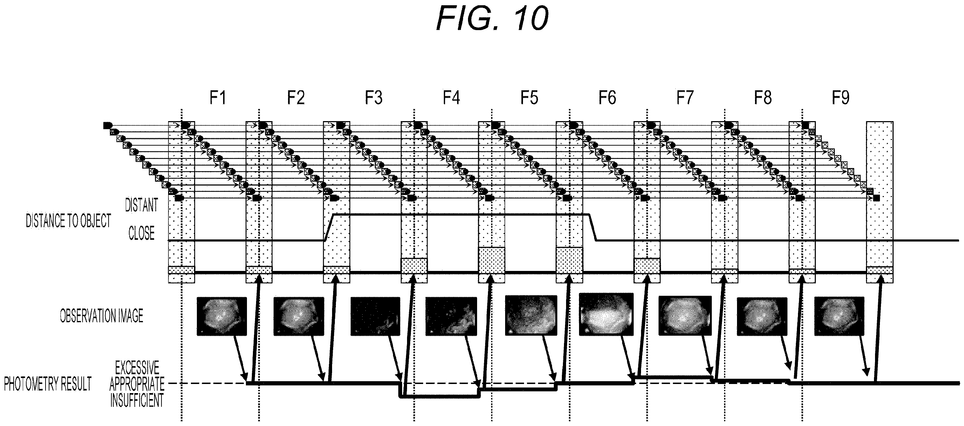

Fig. 10 is a diagram illustrating a state (example) of a dimming control process according to Control Example 1 of the present embodiment and an observation image (example) acquired in each frame. Note that, in Fig. 10, a distance between the endoscope distal tip and the object changes in a similar manner for comparison with the general dimming control process (Fig. 9).

Unlike the general dimming control process, Control Example 1 is a dimming control process of performing globally weak preliminary light emission (continuous light). A value (total value) of light emission time × light emission level of weak preliminary light emission (continuous light) in Control Example 1 is sufficiently smaller than a value of light emission time × light emission level of strong light emission in the pseudo global exposure period. The preliminary light emission is light emission at such an intensity that an undesirable event (for example, distortion or artifacts (such as scanning line noise)) caused by the rolling shutter can be ignored due to light emission (preliminary light emission), and such an intensity that light emission (preliminary light emission) can be recognized when the endoscope distal tip suddenly approaches the object. Specifically, the value of the light emission time of the preliminary light emission × the light emission level may be 10% or less, preferably 2% or less, and more preferably 1% or less of the value of the light emission time of the strong light emission × the light emission level.

In the dimming control process according to Control Example 1 (continuous preliminary light emission), as illustrated in Fig. 10, in a case where the endoscope distal tip is moved away from the object, a process similar to the general dimming control process described above (see Fig. 9) is performed. That is, for example, the frames F1 and F2 can be observed with an appropriate light amount (a photometry result is appropriate). In a pseudo global exposure period between the frame F2 and a frame F3, since the endoscope distal tip is suddenly moved away from an object, an amount of irradiation light to the object is insufficient, and an observation image of the frame F3 is dark. A light amount at the time of acquiring the observation image of the frame F3 is dominated by a light amount from light emission (strong light emission) in the pseudo global exposure period between the previous frame (frame F2) and the current frame (frame F3). Thus, in a case where a distance to the object changes after the pseudo global exposure period, light emission (dimming control) following the change in the distance is performed (reflected) in the next frame (frame F4). In the pseudo global period between the frame F3 and the frame F4, the dimming control is executed stepwise on the basis of the photometry result (light emission control (change of a light emission profile)) is performed stepwise according to the lapse of time (in order of the frames F4, F5, and F6), an appropriate light amount is determined (light amount increase control), and the object is irradiated with an appropriate amount of light. An observation image with an appropriate light amount can be acquired in the frame F5 after the endoscope distal tip is moved away from the object (after being moved away between F2 and F3). Note that the photometry result may be a photometric value of the central portion of the observation image or an average value of the photometric values of all the pixels.

In the pseudo global exposure period between the frame F5 and the frame F6, since a distance between the endoscope distal tip and the object is the same as that in the previous frame F5, light emission is performed in the same profile as that of light emission in the pseudo global exposure period between the frame F4 and the frame F5. However, in the pixel readout period (rolling shutter period) for the frame F6, weak preliminary light emission is performed, and thus, in a case where the endoscope distal tip suddenly approaches the object, a change in brightness of the observation image can be ascertained (approach to the object can be detected). Therefore, the observation image obtained in the frame F6 is brighter than the observation image obtained in the frame F5. That is, the influence of the excessive light amount due to the rapid approach of the endoscope distal tip to the object in the pixel readout period (rolling shutter period) for the frame F6 immediately appears in the observation image. Therefore, when an observation image in the next frame F7 is acquired, a dimming control process reflecting the excess light amount can be executed, and a process of lowering a level of the strong light emission in the pseudo global exposure period between the frame F6 and the frame F7 is performed. However, the light emission level is lowered stepwise so that the brightness changes more naturally for the operator, instead of being lowered rapidly. For example, as illustrated in Fig. 10, the light emission amount in the pseudo global exposure period between the frame F6 and the frame F7 can be first reduced to a predetermined value, and the light emission amount can be further reduced to a predetermined value in the next pseudo global exposure period (between the frame F7 and the frame F8). A control amount (for example, a lowering width) for the light emission amount may be a constant value, or a light emission profile may be determined on the basis of the degree of excess with respect to an appropriate value for a photometry result.

As described above, according to the dimming control process of Control Example 1, the weak preliminary light emission (continuous light) is performed in the pixel readout period (rolling shutter period), and thus, in response to changes of imaging conditions (for example, rapid approach of the endoscope distal tip to the object) in the pixel readout period, the dimming control can be performed one frame earlier than that in the general dimming control process, and the exposure level can be brought close to an appropriate exposure level (an appropriate photometry result can be acquired early).

### (ii) Control Example 2

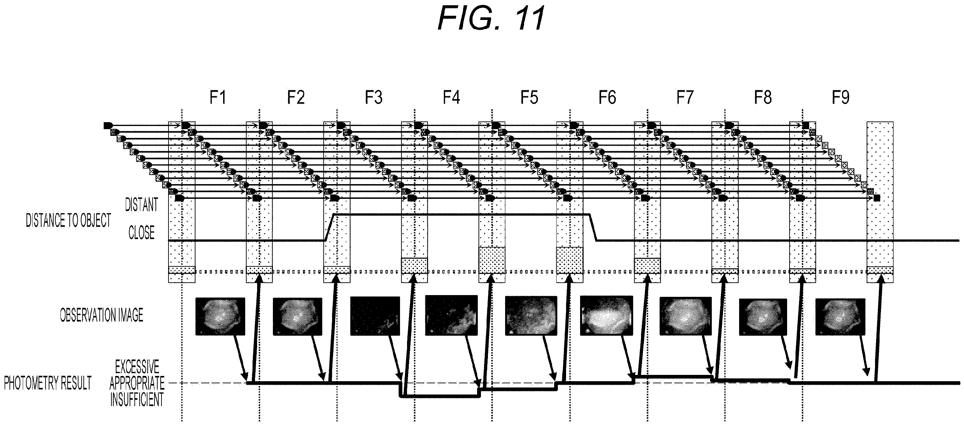

Fig. 11 is a diagram illustrating a state (example) of a dimming control process according to Control Example 2 of the present embodiment and an observation image (example) acquired in each frame. Note that, in Fig. 11, a distance between the endoscope distal tip and the object changes in a similar manner for comparison with the general dimming control process (Fig. 9).

Unlike the general dimming control process, Control Example 2 is a dimming control process performed through globally weak preliminary light emission (pulse light). A value of light emission time × light emission level of weak preliminary light emission (pulse light) in Control Example 2 is sufficiently smaller than a value of light emission time × light emission level of strong light emission in the pseudo global exposure period, similarly to Control Example 1. The preliminary light emission is light emission at such an intensity that an undesirable event (for example, distortion or artifacts (such as scanning line noise)) caused by the rolling shutter can be ignored due to light emission (preliminary light emission), and such an intensity that light emission (preliminary light emission) can be recognized when the endoscope distal tip suddenly approaches the object. Specifically, the value of the light emission time of the preliminary light emission × the light emission level may be 10% or less, preferably 2% or less, and more preferably 1% or less of the value of the light emission time of the strong light emission × the light emission level. In Control Example 2, the pulse light is used for weak preliminary light emission, but a frequency of the pulse is set to be higher by a predetermined value or more. For example, a frequency of the pulse light is set such that an observation image does not have a stripe shape and the entire image becomes bright (whitish) when the endoscope distal tip approaches the object.

Since content and effects of the dimming control process according to Control Example 2 are similar to those of the dimming control process according to Control Example 1 described above, the description thereof will be omitted.

### (iii) Control Example 3

Fig. 12 is a diagram illustrating a state (example) of a dimming control process according to Control Example 3 of the present embodiment and an observation image (example) acquired in each frame. Note that, in Fig. 12, a distance between the endoscope distal tip and the object changes in a similar manner for comparison with the general dimming control process (Fig. 9).

Control Example 3 is a dimming control process of performing weak preliminary light emission (continuous light) only in a predetermined period of a pixel readout period (rolling shutter period) (for example, the last predetermined period of the rolling shutter period (or the predetermined period immediately before the next pseudo global exposure period)).

In the dimming control process according to Control Example 3 (the same applies to Control Example 4 that will be described later), only a predetermined region at the lower part of the screen is subjected to double exposure due to preliminary light emission when approaching the object, as compared with Control Examples 1 and 2 (in a case where preliminary light emission is performed over the entire pixel read (rolling shutter) period). Thus, according to Control Example 3, the discomfort caused by the double exposure to an endoscope user can be reduced, and when the preliminary light emission period is adjusted, a band-shaped line due to the double exposure can be limited to a region that is not noticeable in the observation image. For example, when the frame rate is 60 Hz (16.6 ms) and the global exposure period (in Fig. 12, second upper and lower lines in one frame: the line to be overwritten with the electronic mask display and the invalid pixel line) is 2 ms, the remaining 14.6 ms is the valid line reading period. In this case, when the preliminary light emission is performed for 50% = 7.33 ms of the valid line reading period, a boundary of a region that is not set as a screen region to be subjected to the double exposure is the line at the center of the screen, and an operator feels stronger discomfort. Therefore, for example, a) when 25% = 3.67 ms or less, the boundary is a lower portion corresponding to 1/4 of the screen and is less noticeable than the center, b) when 10% = 1.46 ms or less, the boundary is a lower portion corresponding to 1/10 of the screen and is slightly reflected at the edge of the screen, and thus, the operator hardly cares, and c) when the boundary is only several lines (for example, 10 lines or less) in a predetermined region (for example, the lower region) of the observation image, the boundary substantially coincides with the electronic mask boundary, and the operator cannot recognize a clear boundary. In this case, in a case where the total value of the preliminary light emission period × the preliminary light emission level is not sufficiently smaller than the light emission period × the light emission level of the strong light emission in the pseudo global exposure period, the above-described boundary is clearly seen. In the case of the conditions a) and b) described above, the operator can recognize the boundary although the boundary is inconspicuous. Therefore, it is necessary to make the boundary sufficiently smaller than the strong light emission period × the light emission level (for example, the light emission has such an intensity that an undesirable event (for example, distortion or artifacts) caused by the rolling shutter can be ignored due to the preliminary light emission, and such an intensity that light emission (preliminary light emission) can be recognized in a case where the endoscope distal tip approaches the object rapidly). Specifically, similarly to Control Example 1 and Control Example 2, the value of the light emission time × the light emission level of the preliminary light emission may be 10% or less, preferably 2% or less, and more preferably 1% or less of the value of the light emission time × the light emission level of the strong light emission. On the other hand, in the case of the condition c), since the operator cannot recognize the boundary, the preliminary light emission intensity can be made equal to (or larger than) the strong light emission intensity. That is, since the preliminary light emission period is short, as a result, a product of the preliminary light emission period and the preliminary light emission intensity tends to be small. However, for example, by instantaneously setting the preliminary light emission to 1000 times the strong light emission intensity, there is no problem even when the preliminary light emission is made larger than the strong light emission period × the light emission level.

To summarize a light amount of preliminary light emission, in the case of a) and b), the light emission profile is controlled such that the total value (area value) of the preliminary light emission period × the preliminary light emission level becomes 10% or less, 2% or less, or 1% of the value (area value) of the light emission period × the light emission level of the strong light emission (main light emission). However, in the case of the above c), such a condition is not imposed, and the preliminary light emission level may be extremely increased in relation to the line read in the observation image (not limited to the condition of 10% or less).

In the dimming control process according to Control Example 3 (continuous preliminary light emission in a part of the pixel readout period), as illustrated in Fig. 12, in a case where the endoscope distal tip is moved away from the object, a process similar to the general dimming control process described above (see Fig. 9) is performed. That is, for example, the frames F1 and F2 can be observed with an appropriate light amount (a photometry result is appropriate). In a pseudo global exposure period between the frame F2 and a frame F3, since the endoscope distal tip is suddenly moved away from an object, an amount of irradiation light to the object is insufficient, and an observation image of the frame F3 is dark. A light amount at the time of acquiring the observation image of the frame F3 is dominated by a light amount from light emission (strong light emission) in the pseudo global exposure period between the previous frame (frame F2) and the current frame (frame F3). Thus, in a case where a distance to the object changes after the pseudo global exposure period, light emission (dimming control) following the change in the distance is performed (reflected) in the next frame (frame F4). Thus, in the pseudo global period between the frame F3 and the frame F4, the dimming control is executed stepwise on the basis of the photometry result (light emission control (change of a light emission profile)) is performed stepwise according to the lapse of time (in order of the frames F4, F5, and F6), an appropriate light amount is determined (light amount increase control), and the object is irradiated with an appropriate amount of light. An observation image with an appropriate light amount can be acquired in the frame F5 after the endoscope distal tip is moved away from the object (after being moved away between F2 and F3).

In the pseudo global exposure period between the frame F5 and the frame F6, since a distance between the endoscope distal tip and the object is the same as that in the previous frame F5, light emission is performed in the same profile as that of light emission in the pseudo global exposure period between the frame F4 and the frame F5. However, in the last predetermined period (for example, a period during which the last k lines (where k = 1 to n: n is an integer obtained by rounding up a value of 1% of the number of valid lines (first decimal place) (example)) are read) in the pixel readout period (rolling shutter period) for the frame F6, weak preliminary light emission (continuous light) is performed. Therefore, in a case where the endoscope distal tip suddenly approaches the object, it is possible to ascertain a change in brightness of the observation image (detect that the endoscope distal tip approaches the object). As illustrated in Fig. 12, in the observation image of the frame F6, a portion (region 1201) formed by pixels read in the period in which the preliminary light emission is performed is whiter (brighter) than other portions. That is, the influence of the excessive light amount due to the rapid approach of the endoscope distal tip to the object in the pixel readout period (rolling shutter period) for the frame F6 immediately appears in a part of the observation image. Therefore, when the observation image in the next frame F7 is acquired, a dimming control process reflecting the excess light amount can be executed, and a process of lowering the strong light emission level in the pseudo global exposure period between the frame F6 and the frame F7 (adjustment to appropriate exposure) is performed. However, the light emission level is lowered stepwise so that the brightness changes more naturally for the operator, instead of being lowered rapidly. For example, as illustrated in Fig. 12, the light emission amount in the pseudo global exposure period between the frame F6 and the frame F7 can be first reduced to a predetermined value, and the light emission amount can be further reduced to a predetermined value in the next pseudo global exposure period (between the frame F7 and the frame F8). A control amount (for example, a lowering width) for the light emission amount may be a constant value, or a light emission profile may be determined on the basis of the degree of excess with respect to an appropriate value for a photometry result.

As described above, according to the dimming control process of Control Example 3, the weak preliminary light emission (continuous light) is performed only in a predetermined period (for example, the last predetermined period of the rolling shutter period (or a predetermined period immediately before the next pseudo global exposure period)) of a part of the pixel readout period (rolling shutter period). Therefore, in response to the change (for example, rapid approach of the endoscope distal tip to the object) in the imaging condition in the pixel readout period, the dimming control can be performed one frame earlier than that in the general dimming control process, and the exposure level can be brought close to an appropriate exposure level (an appropriate photometry result can be acquired early).

### (iv) Control Example 4

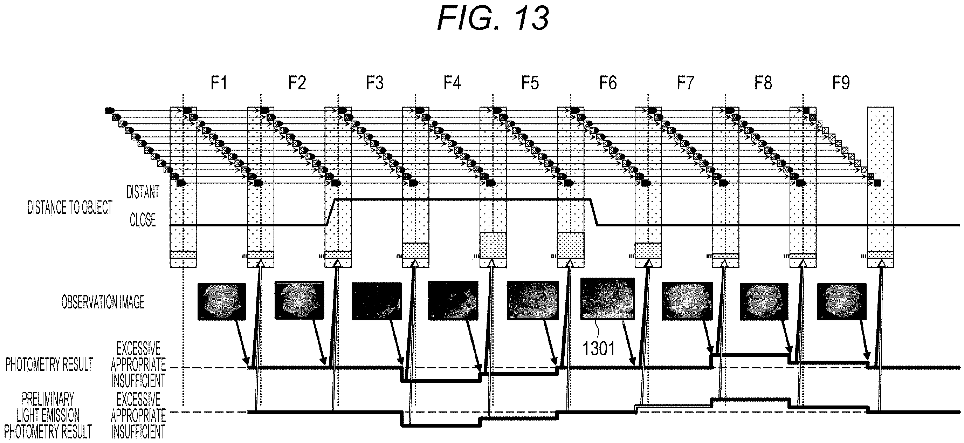

Fig. 13 is a diagram illustrating a state (example) of a dimming control process according to Control Example 4 of the present embodiment and an observation image (example) acquired in each frame. Note that, in Fig. 13, a distance between the endoscope distal tip and the object changes in a similar manner for comparison with the general dimming control process (Fig. 9).

Control Example 4 is a dimming control process of performing weak preliminary light emission (pulse light) only in a predetermined period (for example, the last predetermined period of the rolling shutter period (or the predetermined period immediately before the next pseudo global exposure period)) of the pixel readout period (rolling shutter period). The value of the light emission time × the light emission level of the weak preliminary light emission according to Control Example 4 is sufficiently smaller than the value of the light emission time × the light emission level of the strong light emission in the pseudo global exposure period, similarly to Control Examples 1 to 3. The preliminary light emission is light emission at such an intensity that an undesirable event (for example, distortion or artifacts (such as scanning line noise)) caused by the rolling shutter can be ignored due to light emission (preliminary light emission), and such an intensity that light emission (preliminary light emission) can be recognized when the endoscope distal tip suddenly approaches the object. Specifically, the value of the light emission time of the preliminary light emission × the light emission level may be 10% or less, preferably 2% or less, and more preferably 1% or less of the value of the light emission time of the strong light emission × the light emission level. In Control Example 4, the pulse light is used for the weak preliminary light emission, but a frequency of the pulse is set to be higher by a predetermined value or more. For example, the frequency of the pulse light is set such that a part (region 1301) of the observation image becomes bright (whitish) when the endoscope distal tip approaches the object.

Since content and effects of the dimming control process according to Control Example 4 are similar to those of the dimming control process according to Control Example 3 described above, the description thereof will be omitted.

### <Dimming control process in Control Examples 1 and 2: flowchart>

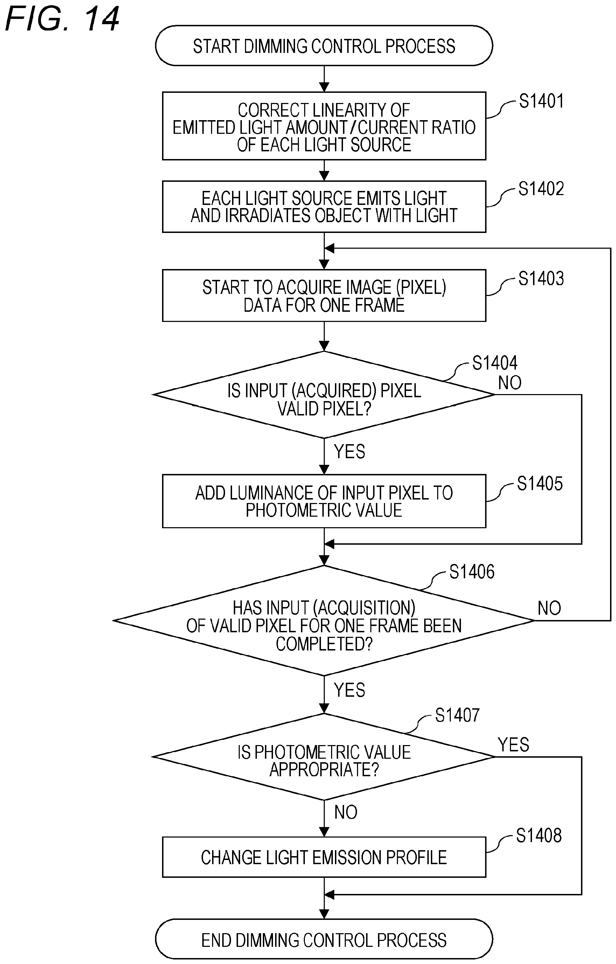

Fig. 14 is a flowchart for describing a dimming control process according to Control Examples 1 and 2 of the present embodiment. Although the process in each of the following steps will be mainly described with the system controller 202 as an operation subject, the present disclosure is not limited thereto, and a control unit (processor) that performs operation control and a calculation process may be separately provided and may be caused to perform the process. The light source control unit 2016 of the light source device 201 may have the functions of the system controller 202. Therefore, the dimming control process may be made a part of the entire operation of the endoscope system 1 or may be made a part of the operation of the light source device 201. In this case, the light source control unit 2016 is a main operation subject of the process in each step.

### (i) Step 1401

The light source control unit 2016 receives a mode selection signal corresponding to an observation mode selected by an operator from the system controller 202 and corrects the linearity of an emitted light amount/current ratio of a light source for each light source (any combination of the green LED 2011 to the UV LED 2015) that is to emit light by using the correction table.

### (ii) Step 1402

The light source control unit 2016 drives each light source with a drive current after the linearity of the emitted light amount/current ratio is corrected to cause each light source to emit light to generate illumination light and irradiates the object with the illumination light. Note that the profile of the strong light emission in the pseudo global exposure period at this time may be a predetermined value (default value), or may employ a light emission profile used in the last operation in the previous use of the endoscope. The light emission profile is determined such that the light emission period × the light emission level of weak preliminary light emission (continuous light or pulse light) in the pixel readout period (rolling shutter period) is 10% or less, 2% or less, or 1% or less of the light emission period (which may be shorter than the pseudo global exposure period) × the light emission level of strong light emission in the pseudo global period as described above (see Figs. 10 and 11).

### (iii) Step 1403

The image sensor (for example, a CMOS sensor) of the imaging unit 103 detects reflected light from the object generated by irradiating the object (observation site) with the illumination light and transmits a captured image signal to the processor 200 via the scope connector circuit 401. The system controller 202 starts to acquire image (each pixel) data for one frame via the photometry unit 203.

### (iv) Step 1404

The system controller 202 determines whether the acquired pixel (input pixel) is a valid pixel (see Fig. 8: a pixel in the valid region of the CMOS sensor). In a case where the acquired pixel is a valid pixel (in a case of YES in step 1404), the process proceeds to step 1405. On the other hand, in a case where the acquired pixel is not a valid pixel (which is an invalid pixel or is a valid pixel but a masked pixel: in a case of NO in step 1404), the process proceeds to step 1406.

### (v) Step 1405

The system controller 202 integrates photometric values (luminance values) of the valid pixels acquired from the photometry unit 203.

### (vi) Step 1406

The system controller 202 determines whether valid pixels for one frame have been acquired. In a case where the acquisition of the valid pixels for one frame has been completed (in a case of YES in step 1406), the process proceeds to step 1407. On the other hand, in a case where the acquisition of the valid pixels for one frame has not been completed yet (in a case of NO in step 1406), the process returns to step 1403.

### (vii) Step 1407

The system controller 202 compares the integrated value of the photometric values for one frame with a predetermined threshold value determined in advance, and determines whether the exposure is appropriate. In a case where it is determined that the photometric values are appropriate (in a case of YES in step 1407), the dimming control process is ended. On the other hand, in a case where it is determined that the photometric values are not appropriate (for example, an excessive light amount due to sudden approach to the object or an insufficient light amount due to movement away from the object) (in a case of NO in step 1407), the process proceeds to step 1408.

### (viii) Step 1408

The system controller 202 changes the light emission profile and transmits the light emission profile to the light source control unit 2016 of the light source device 201. For example, the light emission profile may be changed to reduce or increase the predetermined light amount, or the profile may be determined on the basis of the degree of excess or deficiency with respect to the appropriate value.

A change pattern for the light emission profile may be stored in advance (for example, tabulated) in a memory (not illustrated), and the system controller 202 may determine the light emission profile from a deviation amount (for example, 3 dB excess , a light emission pattern 1, 6 dB excess , a light emission pattern 2, 3 dB deficiency , a light emission pattern 3, ···, and the like) between the measured photometric value and the appropriate photometric value.

As a change of the light emission profile, the light emission level (and/or the light emission period) of the strong light emission in the pseudo global exposure period may be increased or decreased stepwise every frame time (for example, 60 Hz = 16.6 ms) (see F6 , F7 → F8 in Figs. 10 and 11). By doing so, it is possible to avoid a sudden change in brightness, and thus, it is possible to acquire a natural observation image.

### <Dimming control process in Control Examples 3 and 4: Flowchart>

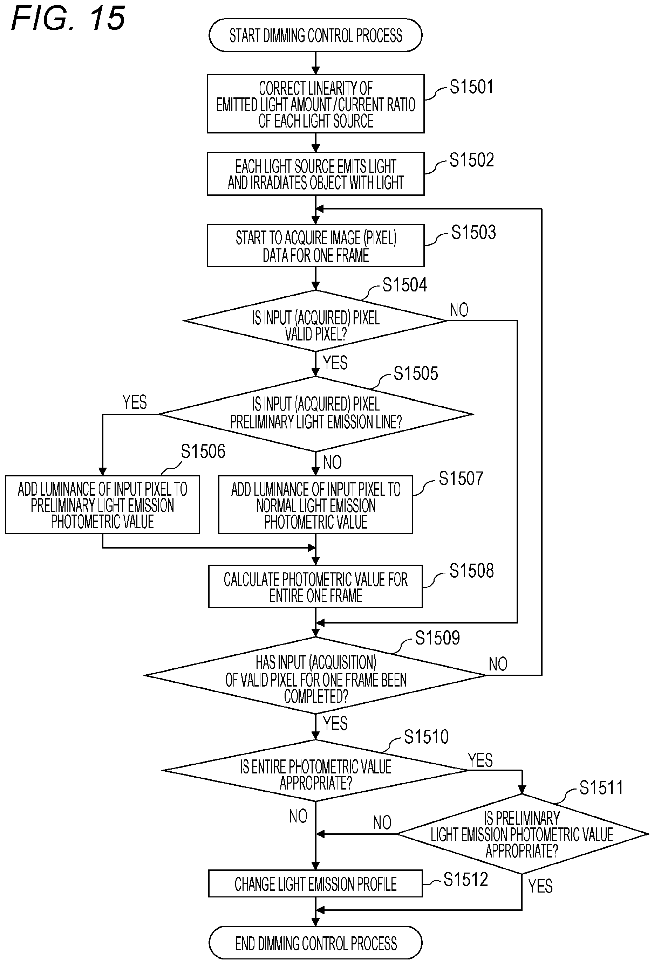

Fig. 15 is a flowchart for describing a dimming control process according to Control Examples 3 and 4 of the present embodiment. Although the process in each of the following steps will be mainly described with the system controller 202 as an operation subject, the present disclosure is not limited thereto, and a control unit (processor) that performs operation control and a calculation process may be separately provided and may be caused to perform the process. The light source control unit 2016 of the light source device 201 may have the functions of the system controller 202. Therefore, the dimming control process may be made a part of the entire operation of the endoscope system 1 or may be made a part of the operation of the light source device 201. In this case, the light source control unit 2016 is a main operation subject of the process in each step.

### (i) Step 1501

The light source control unit 2016 receives a mode selection signal corresponding to an observation mode selected by an operator from the system controller 202 and corrects the linearity of an emitted light amount/current ratio of a light source for each light source (any combination of the green LED 2011 to the UV LED 2015) that is to emit light by using the correction table.

### (ii) Step 1502

The light source control unit 2016 drives each light source with a drive current after the linearity of the emitted light amount/current ratio is corrected to cause each light source to emit light to generate illumination light and irradiates the object with the illumination light. Note that the profile of the strong light emission in the pseudo global exposure period at this time may be a predetermined value (default value), or may employ a light emission profile used in the last operation in the previous use of the endoscope. In Control Example 3 and Control Example 4, weak preliminary light emission (continuous light or pulse light) is performed in a part of the pixel readout period (rolling shutter period) (the last predetermined period of the pixel readout period). As described above, the light emission profile is determined such that the light emission period (which may be shorter than the pseudo global exposure period) × the light emission level of the weak preliminary light emission is 10% or less, 2% or less, or 1% or less of the light emission period × the light emission level of the strong light emission in the pseudo global period (see Figs. 10 and 11).

### (iii) Step 1503

The image sensor (for example, a CMOS sensor) of the imaging unit 103 detects reflected light from the object generated by irradiating the object (observation site) with the illumination light and transmits a captured image signal to the processor 200 via the scope connector circuit 401. The system controller 202 starts to acquire image (each pixel) data for one frame via the photometry unit 203.

### (iv) Step 1504

The system controller 202 determines whether the acquired pixel (input pixel) is a valid pixel (see Fig. 8: a pixel in the valid region of the CMOS sensor). In a case where the acquired pixel is a valid pixel (in a case of YES in step 1504), the process proceeds to step 1505. On the other hand, when the acquired pixel is not a valid pixel (which is an invalid pixel or is a valid pixel but a masked pixel: in a case of NO in step 1504), the process proceeds to step 1509.

### (v) Step 1505

The system controller 202 determines whether the acquired (input) valid pixel is a pixel of the preliminary light emission line. A readout line number and an irradiation timing of weak preliminary light emission in the frame (image) are determined in advance. For example, information regarding a line number and an irradiation body mig is stored in an internal memory of the system controller 202 or a memory (not illustrated). Thus, the system controller 202 can determine in which line weak preliminary light emission is performed.

In a case where it is determined that the acquired pixel is a pixel configuring the preliminary light emission line (in a case of YES in step 1505), the process proceeds to step 1506. On the other hand, in a case where it is determined that the acquired pixel is not related to the preliminary light emission line (in a case of NO in step 1505), the process proceeds to step 1507.

### (vi) Step 1506

The system controller 202 integrates the luminance values of the pixels in the line where weak preliminary light emission is performed to obtain preliminary light emission photometric values (since the preliminary light emission line is also affected by the strong light emission in the pseudo global exposure period, a photometric value is obtained through the strong light emission + the weak preliminary light emission). That is, the luminance value of each pixel configuring the line related to the preliminary light emission is integrated pixel by pixel, and the preliminary light emission photometric value for one frame is finally calculated. The preliminary light emission photometric value for one frame is stored in an internal memory of the system controller 202 or a memory (not illustrated).

### (vii) Step 1507

The system controller 202 integrates luminance values of input pixels other than the preliminary light emission line, and calculates a normal light emission photometric value through strong light emission in the pseudo global exposure period. That is, the luminance value of each pixel configuring the line other than the preliminary light emission line is integrated pixel by pixel, and a normal light emission photometric value for one frame is finally calculated. Note that the normal light emission photometric value for one frame is stored in an internal memory of the system controller 202 or a memory (not illustrated).

### (viii) Step 1508

The system controller 202 adds the preliminary light emission photometric value calculated in step 1506 and the normal light emission photometric value calculated in step 1507 to calculate a photometric value (provisional) for the entire one frame.

### (ix) Step 1509

The system controller 202 determines whether valid pixels for one frame have been acquired. In a case where the acquisition of the valid pixels for one frame has been completed (in a case of YES in step 1509), the process proceeds to step 1510. On the other hand, in a case where the acquisition of the valid pixels for one frame has not been completed yet (in a case of NO in step 1509), the processing returns to step 1503. In a case of YES in step 1509, the photometric value (provisional) for the entire one frame obtained in step 1508 is a photometric value (confirmation) for the entire one frame.

### (x) Step 1510

The system controller 202 compares the photometric value (confirmation) for the entire one frame with a predetermined threshold value (overall photometric threshold value), and determines whether or not the photometric value is appropriate. Note that the overall photometric threshold value may be a value having a predetermined width (range).

In a case where it is determined that the photometric value (confirmation) of the entire one frame is appropriate (in a case of YES in step 1510), the process proceeds to step 1511. On the other hand, when it is determined that the photometric value (confirmation) of the entire one frame is not appropriate (in a case of NO in step 1510), the process proceeds to step 1512.

### (xi) Step 1511

The system controller 202 acquires the preliminary light emission photometric value in the frame, compares the preliminary light emission photometric value with a predetermined threshold value (preliminary light emission threshold value), and determines whether or not the preliminary light emission photometric value is appropriate. Note that the preliminary light emission threshold value may be a value having a predetermined width (range), similarly to the overall photometric threshold value.

In a case where it is determined that the preliminary light emission photometric value is appropriate (in a case of YES in step 1511), the dimming control process is ended. On the other hand, in a case where it is determined that the preliminary light emission photometric value is not appropriate (in a case of NO in step 1511), the process proceeds to step 1512.

### (xii) Step 1512

The system controller 202 changes the light emission profile and transmits the light emission profile to the light source control unit 2016 of the light source device 201. For example, the light emission profile may be changed to reduce or increase the predetermined light amount, or the profile may be determined on the basis of the degree of excess or deficiency with respect to the appropriate value.

A change pattern for the light emission profile may be stored in advance (for example, tabulated) in a memory (not illustrated), and the system controller 202 may determine the light emission profile from a deviation amount (for example, 3 dB excess → the light emission pattern 1, 6 dB excess → the light emission pattern 2, 3 dB deficiency → the light emission pattern 3, ···, and the like) between the measured photometric value and the appropriate photometric value.

As a change of the light emission profile, the light emission level (and/or the light emission period) of the strong light emission in the pseudo global exposure period may be increased or decreased stepwise every frame time (for example, 1/30 seconds) (see F6 → F7 → F8 in Figs. 12 and 13). By doing so, it is possible to avoid a sudden change in brightness, and thus, it is possible to acquire a natural observation image.

### <Effects of present embodiment>

According to the present embodiment, an exposure component (a luminance component due to weak preliminary light emission) in a case where the endoscope distal tip (image sensor) suddenly approaches an object during the rolling shutter period is added to a photometry result. Considering an intensity of light, since a luminance component due to strong light emission (main light emission) in the pseudo global exposure period is overwhelmingly stronger than a luminance component due to weak preliminary light emission in the rolling shutter period, the luminance component due to the strong light emission is dominant in observation image formation (here, the reason why the light emission profile of the strong light emission is determined is that a distance between the image sensor and the object is longer than that at the time of the sudden approach and is thus appropriate). On the other hand, when weak preliminary light emission is being performed and the image sensor is rapidly approaching the object, the entire observation image (Control Examples 1 and 2) or a part of the observation image (a lower portion of the observation image: Control Examples 3 and 4) becomes whitish, and a photometry result due to weak preliminary light emission immediately appears in the observation image, and it is possible to immediately detect that a light amount is excessive. Therefore, according to the dimming control process of the present embodiment, the light amount control can be executed in response to a change during the rolling shutter period (pixel readout period) quickly. In Control Examples 1 and 2 and Control Examples 3 and 4, in the case of the above a) and b), the weak preliminary light emission is controlled to be 10% or less (preferably 2% or less, and more preferably 1% or less) of the main light emission with respect to the value of the light emission period × the light emission level. Thus, it is possible to avoid the occurrence of distortion and artifacts caused by the rolling shutter of the image sensor. In the case of the above c) in Control Examples 3 and 4, since the preliminary light emission period is limited to a period in which several lines of the lower portion of the observation image are read out as formation pixels, even when the light emission level of the preliminary light emission is set to be higher than the light emission level of the main light emission, it is possible to quickly detect the presence or absence of the excessive light amount in the observation image without giving discomfort to an operator.

### <Specified matters of present disclosure>

### (1) Specified matter 1

A light source device that generates illumination light to be applied to an object, the light source device including:
a plurality of semiconductor light emitting elements that emit pieces of light having different wavelength bands; and
a control unit that controls a light emission profile of the plurality of semiconductor light emitting elements and drives the plurality of semiconductor light emitting elements, in which
the control unit controls the light emission profile of the plurality of semiconductor light emitting elements such that main light emission is performed in a pseudo global exposure period of an image sensor and preliminary light emission is performed in at least a part of a pixel readout period of the image sensor, and
a light emission level of the preliminary light emission is lower than a light emission level of the main light emission.

### (2) Specified matter 2

The light source device according to the specified matter 1, in which
a total value of a light emission period × a light emission level of the preliminary light emission is sufficiently smaller than a value of a light emission period × a light emission level of the main light emission such that noise caused by a rolling shutter of the image sensor is ignorable.

### (3) Specified matter 3

The light source device according to the specified matter 1 or 2, in which
the control unit controls a total value of a light emission period × a light emission level of the preliminary light emission to be 10% or less of a value of a light emission period × a light emission level of the main light emission.

### (4) Specified matter 4

The light source device according to any one of the specified matters 1 to 3, in which
the control unit controls the light emission profile such that the preliminary light emission is configured by using continuous light or pulse light over the entire pixel readout period.

### (5) Specified matter 5

The light source device according to any one of the specified matters 1 to 4, in which
the control unit compares a photometric value for one frame of an observation image of the object with a first threshold value, and determines whether or not to change the light emission profile.

### (6) Specified matter 6

The light source device according to the specified matter 5, in which
the control unit changes the light emission profile such that the light emission level of the main light emission is lowered stepwise over a plurality of frames in a case where the photometric value is greater than the first threshold value and a light amount is excessive.

### (7) Specified matter 7

The light source device according to the specified matter 1 or 2, in which
the control unit controls the light emission profile such that the preliminary light emission is performed by using continuous light or pulse light in the pixel readout period of pixels configuring 25% or less of a lower portion of an observation image of the object.

### (8) Specified matter 8

The light source device according to the specified matter 7, in which
the control unit controls a total value of a light emission period × a light emission level of the preliminary light emission to be 10% or less of a value of a light emission period × a light emission level of the main light emission.

### (9) Specified matter 9

The light source device according to the specified matter 1 or 2, in which
the control unit controls the light emission profile such that the preliminary light emission is performed by using continuous light or pulse light during the pixel readout period of pixels configuring a predetermined number of lines of an observation image of the object.

### (10) Specified matter 10

The light source device according to the specified matter 9, in which
the control unit controls the light emission profile such that the preliminary light emission is performed by using the continuous light or the pulse light during the pixel readout period of pixels configuring a final predetermined number of lines of the observation image.

### (11) Specified matter 11

The light source device according to any one of the specified matters 7 to 10, in which
the control unit compares a photometric value based on the preliminary light emission in one frame of the observation image of the object with a second threshold value, and determines whether or not to change the light emission profile.

### (12) Specified matter 12

The light source device according to the specified matter 11, in which
the control unit changes the light emission profile such that the light emission level of the main light emission is lowered stepwise over a plurality of frames in a case where the photometric value is greater than the second threshold value and a light amount is excessive.

### (13) Specified matter 13

An endoscope system in which an endoscope is inserted into an observation target and an image of an object is acquired, the endoscope system including:
a plurality of semiconductor light emitting elements that emit pieces of light having different wavelength bands;
an image sensor that irradiates the object with illumination light and detects reflected light from the object to generate an image signal;
a processor that processes the image signal to generate the image of the object and displays the image on a monitor; a main control unit that generates a control signal for controlling a light emission profile of the plurality of semiconductor light emitting elements on the basis of the image signal; and
a light source control unit that receives the control signal from the main control unit and drives the plurality of semiconductor light emitting elements with a drive signal according to the light emission profile, in which
the main control unit controls the light emission profile of the plurality of semiconductor light emitting elements such that main light emission is performed in a pseudo global exposure period of the image sensor and preliminary light emission is performed in at least a part of a pixel readout period of the image sensor, and
a light emission level of the preliminary light emission is lower than a light emission level of the main light emission.

### (14) Specified matter 14

The endoscope system according to the specified matter 13, in which
a total value of a light emission period × a light emission level of the preliminary light emission is sufficiently smaller than a value of a light emission period × a light emission level of the main light emission such that noise caused by a rolling shutter of the image sensor is ignorable.

### (15) Specified matter 15

The endoscope system according to the specified matter 13 or 14, in which
the main control unit controls a total value of a light emission period × a light emission level of the preliminary light emission to be 10% or less of a value of a light emission period × a light emission level of the main light emission.

### (16) Specified matter 16

The endoscope system according to any one of the specified matters 13 to 15, in which
the main control unit controls the light emission profile such that the preliminary light emission is configured by using continuous light or pulse light over the entire pixel readout period.

### (17) Specified matter 17

The endoscope system according to any one of the specified matters 13 to 16, in which
the main control unit compares a photometric value for one frame of an observation image of the object with a first threshold value, and determines whether or not to change the light emission profile.

### (18) Specified matter 18

The endoscope system according to the specified matter 17, in which
the main control unit changes the light emission profile such that the light emission level of the main light emission is lowered stepwise over a plurality of frames in a case where the photometric value is greater than the first threshold value and a light amount is excessive.

### (19) Specified matter 19

The endoscope system according to the specified matter 13 or 14, in which
the main control unit controls the light emission profile such that the preliminary light emission is performed by using continuous light or pulse light in the pixel readout period of pixels configuring 25% or less of a lower portion of an observation image of the object.

### (20) Specified matter 20

The endoscope system according to the specified matter 19, in which
the main control unit controls a total value of a light emission period × a light emission level of the preliminary light emission to be 10% or less of a value of a light emission period × a light emission level of the main light emission.

### (21) Specified matter 21

The endoscope system according to the specified matter 13 or 14, in which
the main control unit controls the light emission profile such that the preliminary light emission is performed by using continuous light or pulse light during the pixel readout period of pixels configuring a predetermined number of lines of an observation image of the object.

### (22) Specified matter 22

The endoscope system according to the specified matter 21, in which
the main control unit controls the light emission profile such that the preliminary light emission is performed by using the continuous light or the pulse light during the pixel readout period of pixels configuring a final predetermined number of lines of the observation image.

### (23) Specified matter 23

The endoscope system according to any one of the specified matters 19 to 22, in which
the main control unit compares a photometric value based on the preliminary light emission in one frame of the observation image of the object with a second threshold value, and determines whether or not to change the light emission profile.

### (24) Specified matter 24

The endoscope system according to the specified matter 23, in which
the main control unit changes the light emission profile such that the light emission level of the main light emission is lowered stepwise over a plurality of frames in a case where the photometric value is greater than the second threshold value and a light amount is excessive.

### <Others>

The above-described functions of the present embodiment can also be realized by software program codes. In this case, a storage medium recording the program codes is provided to a system or a device, and a computer (or CPU, MPU, etc.) of the system or the device reads the program codes stored in the storage medium. In this case, the program codes read from the storage medium realize the functions of the above-described embodiments, and the program codes and the storage medium storing the program codes are configured to achieve the present disclosure. Examples of the storage medium applicable for supplying such program codes include a flexible disk, CD-ROM, DVD-ROM, hard disk, optical disk, magneto-optical disk, CD-R, magnetic tape, nonvolatile memory card, or ROM.

Moreover, it is allowable to have a configuration in which an operating system (OS) running on the computer performs some or all of actual processes on the basis of the instructions of the program codes, and the functions of the above-described embodiments are realized by the processes. It is also allowable to have a configuration in which the program codes read from the storage medium are first written in the memory on the computer, and thereafter the computer CPU or the like performs some or all of the actual processes on the basis of the instruction of the program codes, so as to realize the functions of the above-described embodiments through the processes.

The program codes of software realizing the functions of the present embodiment may be distributed via a network and stored in storage means such as a hard disk or a memory of a system or a device or a storage medium such as a CD-RW or a CD-R, and a computer (or a CPU or an MPU) of the system or the device may read and execute the program codes stored in the storage means or the storage medium at the time of use.

Finally, the processes and techniques described herein are not inherently related to any particular device and can be implemented by any suitable combination of components. Various types of general purpose devices can be used in accordance with the procedures described herein. It may prove useful to build a dedicated device to execute the steps in the method described herein. Various forms can be formed by appropriately combining a plurality of components disclosed in the present embodiment. For example, some constituents may be deleted from all the constituents described in the present embodiment. Constituents of different embodiments may be appropriately combined with each other. Although the present disclosure has been described in connection with specific examples, these should not be limited in all respects. A person having ordinary knowledge in the technical field (person skilled in the art) may understand that there are many combinations of hardware, software, and firmware suitable for implementing the technology of the present disclosure. For example, the software in description can be implemented in a wide range of programs or script languages such as assembler, C/C++, perl, Shell, PHP, Java (registered trademark).

In the above-described embodiment, control lines and information lines are considered to be necessary for explanation, and these are not necessarily illustrating control lines and information lines associated with the product. All the constituents may be connected to each other.

### Reference Signs List

- 1: Endoscope system
- 100: Endoscope device
- 103: Imaging unit
- 200: Processor
- 201: Light source device
- 2011: Green LED
- 2012: Blue LED
- 2013: Red LED
- 2014: Amber LED
- 2015: UV LED
- 2016: Light source control unit
- 2017, 2018: Cross prism
- 202: System controller
- 203: Photometry unit
- 300: Monitor

## Claims

1. A light source device that generates illumination light to be applied to an object, the light source device comprising:
a plurality of semiconductor light emitting elements that emit pieces of light having different wavelength bands; and
a control unit that controls a light emission profile of the plurality of semiconductor light emitting elements and drives the plurality of semiconductor light emitting elements, wherein
the control unit controls the light emission profile of the plurality of semiconductor light emitting elements such that main light emission is performed in a pseudo global exposure period of an image sensor and preliminary light emission is performed in at least a part of a pixel readout period of the image sensor, and
a light emission level of the preliminary light emission is lower than a light emission level of the main light emission.

2. The light source device according to claim 1, wherein
a total value of a light emission period × a light emission level of the preliminary light emission is sufficiently smaller than a value of a light emission period × a light emission level of the main light emission such that noise caused by a rolling shutter of the image sensor is ignorable.

3. The light source device according to claim 1, wherein
the control unit controls a total value of a light emission period × a light emission level of the preliminary light emission to be 10% or less of a value of a light emission period × a light emission level of the main light emission.

4. The light source device according to claim 1, wherein
the control unit controls the light emission profile such that the preliminary light emission is configured by using continuous light or pulse light over the entire pixel readout period.

5. The light source device according to claim 1, wherein
the control unit compares a photometric value for one frame of an observation image of the object with a first threshold value, and determines whether or not to change the light emission profile.

6. The light source device according to claim 5, wherein
the control unit changes the light emission profile such that the light emission level of the main light emission is lowered stepwise over a plurality of frames in a case where the photometric value is greater than the first threshold value and a light amount is excessive.

7. The light source device according to claim 1, wherein
the control unit controls the light emission profile such that the preliminary light emission is performed by using continuous light or pulse light in the pixel readout period of pixels configuring 25% or less of a lower portion of an observation image of the object.

8. The light source device according to claim 7, wherein
the control unit controls a total value of a light emission period × a light emission level of the preliminary light emission to be 10% or less of a value of a light emission period × a light emission level of the main light emission.

9. The light source device according to claim 1 or 2, wherein
the control unit controls the light emission profile such that the preliminary light emission is performed by using continuous light or pulse light during the pixel readout period of pixels configuring a predetermined number of lines of an observation image of the object.

10. The light source device according to claim 9, wherein
the control unit controls the light emission profile such that the preliminary light emission is performed by using the continuous light or the pulse light during the pixel readout period of pixels configuring a final predetermined number of lines of the observation image.

11. The light source device according to claim 7, wherein
the control unit compares a photometric value based on the preliminary light emission in one frame of the observation image of the object with a second threshold value, and determines whether or not to change the light emission profile.

12. The light source device according to claim 11, wherein
the control unit changes the light emission profile such that the light emission level of the main light emission is lowered stepwise over a plurality of frames in a case where the photometric value is greater than the second threshold value and a light amount is excessive.

13. An endoscope system in which an endoscope is inserted into an observation target and an image of an object is acquired, the endoscope system comprising:
a plurality of semiconductor light emitting elements that emit pieces of light having different wavelength bands;
an image sensor that irradiates the object with illumination light and detects reflected light from the object to generate an image signal;
a processor that processes the image signal to generate the image of the object and displays the image on a monitor (300);
a main control unit that generates a control signal for controlling a light emission profile of the plurality of semiconductor light emitting elements on the basis of the image signal; and
a light source control unit (2016) that receives the control signal from the main control unit and drives the plurality of semiconductor light emitting elements with a drive signal according to the light emission profile, wherein
the main control unit controls the light emission profile of the plurality of semiconductor light emitting elements such that main light emission is performed in a pseudo global exposure period of the image sensor and preliminary light emission is performed in at least a part of a pixel readout period of the image sensor, and
a light emission level of the preliminary light emission is lower than a light emission level of the main light emission.

14. The endoscope system according to claim 13, wherein
a total value of a light emission period × a light emission level of the preliminary light emission is sufficiently smaller than a value of a light emission period × a light emission level of the main light emission such that noise caused by a rolling shutter of the image sensor is ignorable.

15. The endoscope system according to claim 13, wherein
the main control unit controls a total value of a light emission period × a light emission level of the preliminary light emission to be 10% or less of a value of a light emission period × a light emission level of the main light emission.

16. The endoscope system according to claim 13, wherein
the main control unit controls the light emission profile such that the preliminary light emission is configured by using continuous light or pulse light over the entire pixel readout period.

17. The endoscope system according to claim 13, wherein
the main control unit compares a photometric value for one frame of an observation image of the object with a first threshold value, and determines whether or not to change the light emission profile.

18. The endoscope system according to claim 17, wherein
the main control unit changes the light emission profile such that the light emission level of the main light emission is lowered stepwise over a plurality of frames in a case where the photometric value is greater than the first threshold value and a light amount is excessive.

19. The endoscope system according to claim 13, wherein
the main control unit controls the light emission profile such that the preliminary light emission is performed by using continuous light or pulse light in the pixel readout period of pixels configuring 25% or less of a lower portion of an observation image of the object.

20. The endoscope system according to claim 19, wherein
the main control unit controls a total value of a light emission period × a light emission level of the preliminary light emission to be 10% or less of a value of a light emission period × a light emission level of the main light emission.

21. The endoscope system according to claim 13, wherein
the main control unit controls the light emission profile such that the preliminary light emission is performed by using continuous light or pulse light during the pixel readout period of pixels configuring a predetermined number of lines of an observation image of the object.

22. The endoscope system according to claim 21, wherein
the main control unit controls the light emission profile such that the preliminary light emission is performed by using the continuous light or the pulse light during the pixel readout period of pixels configuring a final predetermined number of lines of the observation image.

23. The endoscope system according to claim 19, wherein
the main control unit compares a photometric value based on the preliminary light emission in one frame of the observation image of the object with a second threshold value, and determines whether or not to change the light emission profile.

24. The endoscope system according to claim 23, wherein
the main control unit changes the light emission profile such that the light emission level of the main light emission is lowered stepwise over a plurality of frames in a case where the photometric value is greater than the second threshold value and a light amount is excessive.
